Europäisches Patentamt

(19) European Patent Office

Office européen des brevets

(11) Publication number: **0 015 075**
**B1**

(12) # EUROPEAN PATENT SPECIFICATION

(45) Date of publication of patent specification: 02.03.83

(51) Int. Cl.³: **A 61 B 5/14, G 01 N 27/48**

(21) Application number: 80300270.8

(22) Date of filing: 30.01.80

(54) Bilumen catheter comprising a polarographic sensor and method of manufacturing it.

(30) Priority: **31.01.79 US 8223**

(43) Date of publication of application:
**03.09.80 Bulletin 80/18**

(45) Publication of the grant of the patent:
**02.03.83 Bulletin 83/9**

(84) Designated Contracting States:
**CH DE FR GB IT NL SE**

(56) References cited:
**DE - A - 2 733 003**
**FR - A - 2 268 265**
**FR - A - 2 276 024**
**GB - A - 1 116 766**
**US - A - 3 912 614**
**US - A - 3 983 864**
**US - A - 4 016 864**
**US - A - 4 041 933**

(73) Proprietor: **MCNEILAB, INC.**
**Camp Hill Road Fort Washington**
**Pennsylvania 19034 (US)**

(72) Inventor: **Parker, Dawood**
**21892 Winnebago**
**El Toro California (US)**

(74) Representative: **Colgan, Stephen James et al,**
**CARPMAELS & RANSFORD 43 Bloomsbury**
**Square**
**London WC1A 2RA. (GB)**

(56) References cited:
**MEDICAL AND BIOLOGICAL ENGINEERING**
**AND COMPUTING, vol. 16, no. 5, September**
**1978, pages 599—600 Stevenage, G. B. D.**
**PARKER et al.: "Catheter-tip electrode for**
**continuous measurement of pO2 and pCO2"**

Courier Press, Leamington Spa, England.

# Bilumen catheter comprising a polarographic sensor and method of manufacturing it

## Field of the invention

This invention relates to electrochemical sensors for blood gases, and particularly to combination sensors, such as for neonatal applications, whereby blood samples may be withdrawn through the gas sensing catheter.

## Background of the invention and prior art

In US—A—3 912 614 to Spracklen et al, issued October 14, 1975 and entitled "Sensor", there is disclosed a monopolar catheter type electrochemical sensor, to be used in conjunction with an external, such as transcutaneous, anode electrode for *in vivo* sensing of blood gas concentration. In accordance with the sensors set forth in that patent, an elongate conductor terminates in a tip defining the electrode, a noble metal of predetermined area, enclosed in an epoxy resin but for the active surface. The assembly is encapsulated in a selectively permeable membrane which allows selected fluid constituents to migrate through the membrane for electrochemical reaction at the electrode.

Electrodes in accordance with the foregoing Spracklen et al. patent are primarily intended for insertion, in catheter fashion, into a vein or an artery, or into cellular tissue. The external electrode is affixed to the body, and the potential difference between the respective electrodes is monitored, thereby yielding representation of concentration of the material (e.g. oxygen concentration in blood) to which the membrane is selectively permeable.

Monopolar polarographic sensors of the type set forth in the aforementioned Spracklen et al. patent are advantageously applied to the monitoring of blood gas conditions in neonates. Especially in the case of premature babies for whom continuous and intensive care is required, it is important periodically to monitor many blood parameters simultaneously, and continuously to monitor a few specific parameters, such as oxygen partial pressure. One conventional procedure entails periodic umbilical insertion of a sampling catheter, whereby blood samples may be withdrawn and analyzed, followed by umbilical insertion of blood gas sensor catheters such as those shown in the Spracklen et al. patent.

The present invention is concerned with providing an improved polarographic sensor, which is suitable for neonatal applications (or other similar applications calling for periodic fluid sample withdrawals) without the need for intermittent withdrawals and insertions of various types of catheter. This may be achieved by use of a dual function catheter: which (a) acts as a polarographic sensor and which (b) may simultaneously be employed to withdraw from or supply fluid to the patient.

Dual function catheters have been described in the literature.

For example, GB—A—1 116 766 describes a bilumen catheter in which one lumen is adapted for use as a polarographic sensor and the other lumen is open at its proximal end. The polarographic sensor is a bipolar device wherein the anode and cathode are wires retained in the lumen at the proximal end by an inert epoxy resin. A permeable membrane is mechanically attached across the end of this lumen. The open lumen is described for use *in vivo* manometric blood pressure monitoring.

US—A—4 041 933 also describes bilumen catheters, incorporating polarographic sensors in one lumen, both monopolar and bipolar. The other lumen is open either at the proximal end of the catheter or to one side, adjacent the proximal end.

Another approach has been in which a tubular catheter is extruded with an electrode/wire conductor embedded in the catheter wall.

Prior designs however involve manufacturing difficulties and ultimate uncertainty as to the isolation of the components of the polarographic sensor from the fluid withdrawal channel (the open lumen).

We have now devised an improved bilumen catheter comprising a polarographic sensor which provides reliable isolation of the electrochemical components for polarography from the open lumen.

## Summary of the present invention

According to a first aspect, the invention provides a bilumen catheter comprising a polarographic sensor, including:

a) an elongate flexible member (101), one end of which is adapted for insertion into a patient, said member (101) defining at least two distinct, longitudinal channels (201, 202) therein and an opening (203) at said one end for communication of one (201) of said channels with ambient patent body fluids;

b) at least one electrical conductor (204) located in the other (202) of said channels, said conductor (204) terminating with a predetermined cross-section (207) at said one end of said member (101);

c) potting means (206) for sealably closing said other channel (202), said potting means (206) enclosing said conductor (204) whilst exposing said predetermined cross-section (207); and

d) selectively-permeable membrane means (208) overlaying at least said exposed predetermined cross-section (207);

characterised in that:
said opening (203) penetrates a side wall of

said member (101) in the region of said one end of said member (101), and said potting means (206) further sealably closes said one channel (201), insulating said membrane means (208) and said conductor (204) from said opening (203) and said one channel (201).

According to a second aspect, the invention provides a method of manufacturing a bilumen catheter comprising a polarographic sensor, comprising:

a) providing a flexible bilumen catheter (101);
b) providing a noble metal cathode wire (204) in a first lumen (202) of said catheter;
c) sealably closing said first lumen (202) at one extremity with an inert material (206), with a terminating portion of said wire of predetermined cross-section (207) remaining exposed, and
d) covering said terminating portion of predetermined cross-section (207) with a selectively-permeable membrane (208);

characterised in that:
said wire (204) terminates at said extremity in a portion which is varnish-insulated (said terminating portion of predetermined cross-section (207) being uninsulated), said inert material (206) additionally closes the second lumen (201) of said catheter, said membrane (208) is provided by dipcoating said extremity whereby said membrane (208) covers the outside walls of said catheter (101) at said extremity and said inert material (206), and wherein a further step is provided of opening (203) the said second lumen (201) to the outside of said catheter (101).

The present invention typically involves a catheter type polarographic electrode assembly wherein an elongate flexible member, adapted for insertion into a patient, carries two distinct longitudinal channels, or lumens, therein. One channel carries an electrical conductor, and the other is open for blood withdrawal or for drug or nutrient infusion. The assembly terminates in the actual polarographic sensor, wherein an epoxy resin seals the end of both lumens, holds the electrode wire, and exposes a predetermined cross-section thereof. The tip is coated with a membrane such as polyhydroxyethylmethacrylate, and the free lumen is opened to the exterior for purposes of fluid exchange.

Description of the drawings
Fig. 1 shows a preferred embodiment of the present invention;
Fig. 2 shows a cross-section of a relevant portion of the embodiment of Fig. 1.

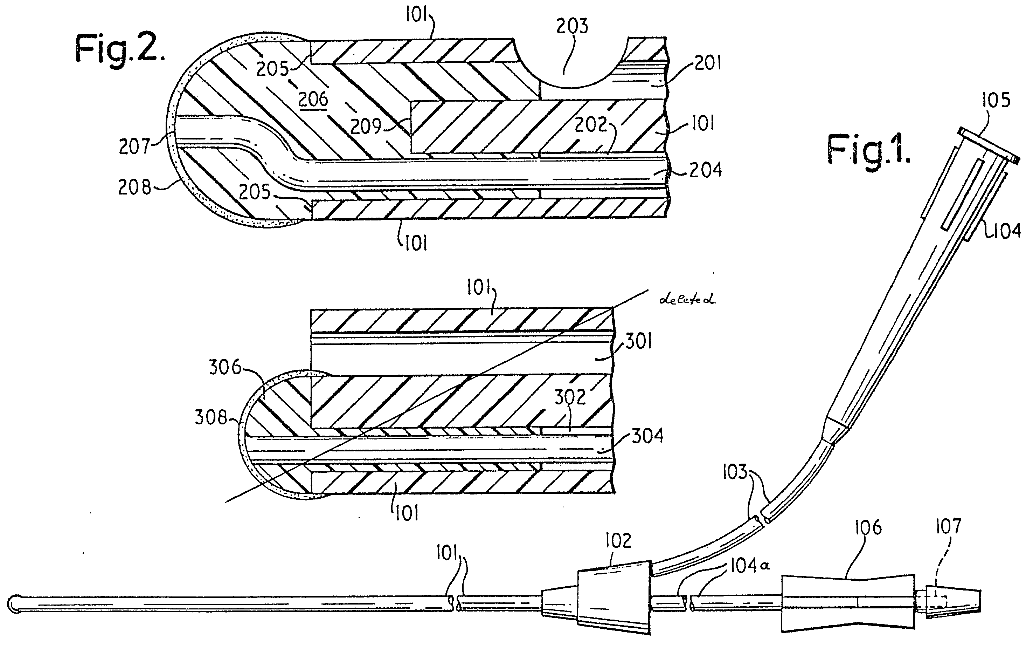

Best mode for carrying out the invention
Referring to Figs. 1 and 2, there is shown a preferred embodiment of the present invention. A bilumen catheter 101 has two separate lumens therein which separate at a junction block 102 into separate conduits 103 and 104a. The former conduit 103 is coupled at junction block 102 with a first, open lumen 201 of the catheter 101, and thereby is useful for withdrawal or sampling of fluids from the tip of catheter 101. The conduit 103 terminates in a suitable connector 104 and port 105, for coupling with infusion/withdrawal apparatus, many types of which are readily available and well-known in the art. Conduit 104a is coupled via junction block 102 to a second lumen 202 of the catheter 101, which carries an electrical conductor 204 from a polarographic sensor 207—208 located at the tip of catheter 101. The conduit 104a includes an electrical insulator block 106 and terminates in a suitable electrical connector 107, whereby the polarographic sensing aspects may be coupled to external signal processing and/or metering equipment, which is also suitably coupled. The signal processing aspects are also connected to a datum electrode (not shown). As is known in the art, this datum (anode) electrode is preferably transcutaneously carried by the patient. Alternatively, as is also known in the art, suitable *in vivo* or subcutaneous datum electrical connections may be established with the patient.

In practice, the catheter 101 is inserted into the patient (e.g. umbilically into a neonate, or intravascularly into a suitable limb of the patient) utilizing apparatus and techniques known in the art (e.g. refer to US—A—3 912 614 to Spracklen et al., or to numerous sources of cannula design and use techniques).

The features of the present invention are clearly manifest from consideration of Fig. 2, which sets forth a cross-sectional view of the tip of the catheter 101 of Fig. 1. In Fig. 2, the catheter 101 defines therein two distinct channels or lumens 201 and 202, the former lumen 201 being opened to form an intravascular port 203 which communicates via catheter 101 to the infusion and sampling port 104 and 105 via junction block 102. The second lumen 202 carries an electrical conductor 204 through the catheter 101 to the insulator 106 and connector 107, and thence to external signal processing and metering apparatus (not shown). In the embodiment of Fig. 2, the conductor 204 extends outwardly beyond the extremity 205 of bilumen catheter 101, and is held there by an inert potting means such as epoxy 206. Pursuant to the embodiment of Fig. 2, the epoxy 206 extends into both lumens 201 and 202 of catheter 101, sealing both whereby conductor 204 is held rigidly and permanently in place, as shown, and whereby intravascular port 203 opens the infusion/sampling lumen 201 to the ambient surroundings of the catheter tip. In accordance with the principles set forth in US—A—3 912 614 to Spracklen et al., a monopolar sensor is formed by covering the tip 207 of conductor 204 (which has a carefully controlled predetermined cross-section) with a selectively permeable membrane 208. Hence, in accordance with the teachings of the Spracklen et al. patent, a gas in solution in the blood, such as oxygen, migrates through membrane 208, and sets up the now well-known polarographic electrochemical reaction in conjunction with the remote datum electrode.

In a preferred mode of constructing the catheter electrode configuration of Fig. 2, the bilumen catheter body 101 is provided and formed as shown, with its central divider portion 209 being recessed backwardly from the extreme terminus 205 to accommodate bending of wire 204 and positioning of the tip 207 generally in the central extreme of the catheter. The conductor 204 is threaded through the lumen 202 and shaped as shown, and the epoxy compound 206 is provided to fill the ends of lumens 201 and 202, and when set, to hold conductor 204 in place as shown. In a preferred construction, the conductor 204 is composed of a noble metal, e.g. silver, which is provided with a coating of varnish insulation at least in the end portion shown in Fig. 2, thereby promoting adhesion of the silver wire 204 with the epoxy resin 206. The active portion, tip 207 of conductor 204, is substantially free of varnish or epoxy coatings, providing full and even contact between the conductor tip 207 and the membrane 208.

Once the epoxy has set, the intravascular port 203 is provided by cutting the side of catheter 101 into lumen 201. A preferred location for the port 203 is substantially at the point of blockage of lumen 201 by the epoxy resin 206, as shown, thereby providing a smooth and relatively laminar flow capability for fluids being infused into the patient via lumen 201, or being withdrawn from the patient through that lumen.

The membrane 208 is preferably composed of polyhydroxyethylmethacrylate, which preferably is formed by multiple dip coatings. In particular, it has been found that several (e.g. four) successive coatings of polyhydroxyethylmethacrylate, with each coating being allowed to dry before application of the next, eliminates formation of air bubbles in the membrane which would lead to sensor malfunctioning, yet provides membranes of the type and thickness useful, as is known in the art, for polarographic applications. In a preferred embodiment, the conductor tip diameter 207 of the silver conductor 204 is 102 $\mu$m (.004 inches), resulting in a current output from the sensor of about $6 \times 10^{-9}$ amperes at a blood oxygen partial pressure of 20.0 kPa (150 millimeters of mercury). At such sensitivities, current output is relatively easy to measure by known means, while at the same time the consumption of oxygen by the sensor is so small that the sensor output is nearly independent of flow velocity of blood past the catheter tip.

It will be noted that the configuration of Fig. 2 locates the cathode of the sensor at the front tip of the catheter, and thus in a region of relatively high blood velocity. Such would not necessarily be the case if the cathode were located at the side of the catheter tip, where blood may be virtually stagnant.

The dip coating technique for formation of the membrane 208 enables the membrane to be coated onto the catheter tip from solution and fall under gravity to form a smooth featureless membrane surface. This configuration contributes to biocompatibility and deposition free operation of the sensor. Intimate contact between the membrane and cathode is also thereby obtained, insuring that the relative movement between these components is not possible, thereby substantially eliminating hydrostatic pressure sensitivity.

In a preferred embodiment, the bilumen catheter material 101 is polyvinyl chloride.

In a useful application of the embodiment of Figs. 1 and 2, the catheter is suitably placed in the patient, and a blood sample is withdrawn via port 203, lumen 201, and withdrawal port 104 and 105. A "one-shot" static measurement/analysis is made with respect to a large variety of blood parameters and blood gases in solution, including $pO_2$. In conjunction with these readings, the current output from sensor 207—208, via conductor 204 and connector 107, is monitored and the sensor system is calibrated. Thereupon, continuous or periodic $pO_2$ measurements may be taken via the sensor 207—208. At any time, nutrient or medicinal materials may be infused into the patient, via lumen 201 and port 203, and any other appropriate blood parameters may be evaluated periodically by sample withdrawal through lumen 201, without the need to displace the catheter 101 or to interrupt $pO_2$ monitoring at the sensor 207—208.

**Claims**

1. A bilumen catheter comprising a polarographic sensor, including:

a) an elongate flexible member (101), one end of which is adapted for insertion into a patient, said member (101) defining at least two distinct, longitudinal channels (201, 202) therein and an opening (203) at said one end for communication of one (201) of said channels with ambient patient body fluids;

b) at least one electrical conductor (204) located in the other (202) of said channels, said conductor (204) terminating with a predetermined cross-section (207) at said one end of said member (101);

c) potting means (206) for sealably closing said other channel (202), said potting means (206) enclosing said conductor (204) whilst exposing said predetermined cross-section (207); and

d) selectively-permeable membrane means (208) overlaying at least said exposed predetermined cross-section (207);

characterised in that:

said opening (203) penetrates a side wall of said member (101) in the region of said one end of said member (101), and said potting means (206) further sealably closes said one channel (201), insulating said membrane means (208) and said conductor (204) from said opening (203) and said one channel (201).

2. A bilumen catheter comprising a polarographic sensor according to Claim 1, characterised in that a central portion (209) of the flexible member (101) which separates said at least two channels (201, 202) is recessed backwardly at said end of said member (101) so to accommodate a bend in said conductor (204) where it leaves said other channel (202) whereby to position the termination of predetermined cross-section (207) at the centre of the end of said flexible member (101).

3. A bilumen catheter comprising a polarographic sensor according to Claim 2, characterised in that said potting means (206) sealably encloses the end of said central portion (209).

4. A bilumen catheter comprising a polarographic sensor according to any of Claims 1 to 3, characterised in that it further includes means (103, 104, 105) for withdrawal of samples of said ambient patient body fluid via said opening (203) and said one channel (201).

5. A method of manufacturing a bilumen catheter comprising a polarographic sensor, comprising:

a) providing a flexible bilumen catheter (101);
b) providing a noble metal cathode wire (204) in a first lumen (202) of said catheter;
c) sealably closing said first lumen (202) at one extremity with an inert material (206), with a terminating portion of said wire of predetermined cross-section (207) remaining exposed, and
d) covering said terminating portion of predetermined cross-section (207) with a selectively-permeable membrane (208);

characterised in that:

said wire (204) terminates at said extremity in a portion which is varnish-insulated (said terminating portion of predetermined cross-section (207) being uninsulated), said inert material (206) additionally closes the second lumen (201) of said catheter, said membrane (208) is provided by dipcoating said extremity whereby said membrane (208) covers the outside walls of said catheter (101) at said extremity and said inert material (206), and wherein a further step is provided of opening (203) the said second lumen (201) to the outside of said catheter (101).

6. A method according to Claim 5, characterised in that said dipcoating comprises application of successive dip and dry cycles employing polyhydroxyethylmethacrylate.

7. A method according to Claim 5 or 6, characterised in that said inert material (206) is an epoxy resin.

8. A method according to any of Claims 5 to 7, characterised in that a central portion (209) of the catheter (101) which separates said two lumens (201, 202) is recessed backwardly at said extremity, and said wire (204) is bent as it leaves said first lumen (202) so positioning the termination of predetermined cross-section (207) at the centre of said extremity of said catheter (101).

**Patentansprüche**

1. Zweikanal-Katheter mit einem polarografischen Sensor, mit:

a) ein mit einem Ende in einen Patienten einführbaren, langgestreckten, flexiblen Gebilde (101), welches wenigstens zwei voneinander getrennte, in Längsrichtung verlaufende Kanäle (201, 202) umgrenzt und an dem einen Ende eine Öffnung (203) hat, über welche einer (201) der Kanäle in Strömungsverbindung mit umgebenden Körperflüssigkeiten des Patienten bringbar ist,
b) wenigstens einem im anderen (202) der Kanäle angeordneten elektrischen Leiter (204), welcher mit einem vorbestimmten Querschnitt (207) an dem einen Ende des Gebildes (101) endet,
c) einer den anderen Kanal (202) abdichtend verschießenden Einbettung (206), welche den Leiter (204) umschließt, dabei jedoch den vorbestimmten Querschnitt (207) unbedeckt läßt, und
d) einem wenigstens den unbedeckten vorbestimmten Querschnitt (207) überdeckenden, selektiv durchlässigen Membranengebilde (208),

dadurch gekennzeichnet, daß:

die Öffnung (203) eine Seitenwand des Gebildes (101) im Bereich des einen Endes desselben durchsetzt, und daß die Einbettung (206) außerdem den einen Kanal (201) abdichtend verschließt und damit das Membranengebilde (208) und den Leiter (204) gegenüber der Offnung (203) und dem einen Kanal (201) isoliert.

2. Zweikanal-Katheter mit polarografischem Sensor nach Anspruch 1, dadurch gekennzeichnet, daß ein die wenigstens zwei Kanäle (201, 202) voneinander trennendes mittleres Teil (209) des flexiblen Gebildes (101) an dem einen Ende des Gebildes (101) rückwärts eingekürzt ist, um einer Biegung des Leiters (204) an seinem Austritt aus dem anderen Kanal (202) Raum zu geben, so daß das einem vorbestimmten Querschnitt (207) aufweisende Ende desselben in der Mitte des Endes des flexiblen Gebildes (101) ausrichtbar ist.

3. Zweikanal-Katheter mit polarografischem Sensor nach Anspruch 2, dadurch gekennzeichnet, daß die Einbettung (206) das Ende des mittleren Teils (209) abdichtend umschließt.

4. Zweikanan-Katheter mit polarografischem Sensor nach wenigstens einem der Ansprüche 1 bis 3, dadurch gekennzeichnet, daß er ferner Einrichtungen (103, 104, 105) für die Entnahme von Proben der umgebenden Körperflüssigkeit des Patienten über die Öffnung (203) und den einen Kanal (201) aufweist.

5. Verfahren zum Herstellen eines Zweikanal-Katheters mit einem polarografischen Sensor, bei welchem

a) ein flexibler Zweikanal-Katheter (101) gefertigt wird,

b) ein Kathodendraht (204) aus Edelmetall in einem ersten Kanal (202) des Katheters angeordnet wird,

c) der erste Kanal (202) an einem Ende mit einem inerten Material (206) abdichtend verschlossen wird, wobei ein einen vorbestimmten Querschnitt (207) aufweisendes Endstück des Drahts unbedeckt bleibt, und

d) das einen vorbestimmten Querschnitt (207) aufweisende Endstück mit einer selektiv durchlässigen Membrane (208) überzogen wird,

dadurch gekennzeichnet, daß:

der Draht (204) an dem Ende in einem mit Lack isolierten Stück ausläuft (wobei das den vorbestimmten Querschnitt (207) aufweisende Endstück nicht isoliert ist), daß das inerte Material (206) zusätzlich den zweiten Kanal (201) des Katheters verschließt, daß die Membrane (208) durch Tauchbeschichtung des Endes gebildet wird, so daß sie die Außenwände des Endes des Katheters (101) sowie das inerte Material (206) überzieht, und daß in einem weiteren Schritt eine den zweiten Kanal (201) mit dem Äußeren des Katheters (101) verbindende Öffnung (203) gebildet wird.

6. Verfahren nach Anspruch 5, dadurch gekennzeichnet, daß die Tauchbeschichtung durch aufeinander folgende Tauch- und Trockenzyklen unter Verwendung von Polyhydroxyäthylmethacrylat bewerkstelligt wird.

7. Verfahren nach Anspruch 5 oder 6, dadurch gekennzeichnet, daß das inerte Material (206) ein Epoxydharz ist.

8. Verfahren nach wenigstens einem der Ansprüche 5 bis 7, dadurch gekennzeichnet, daß ein die beiden Kanäle (201, 202) voneinander trennendes mittleres Teil (209) des Katheters (101) an dem Ende rückwärts eingekürzt ist und daß der Draht (204) an seinem Austritt aus dem ersten Kanal (202) derart gebogen ist, daß sein einen vorbestimmten Querschnitt (207) aufweisendes Ende in der Mitte des Endes des Katheters (101) ausgerichtet ist.

**Revendications**

1. Un cathéter à deux canaux, comprenant un détecteur polarographique, comportant:

a) un organe flexible allongé (101) dont une première extrémité est adaptée pour être introduite dans un patient, ledit organe (101) comportant au moins deux canaux longitudinaux distincts (201, 202) et une ouverture (203) à ladite première extrémité pour faire communiquer un premier (201) desdits canaux avec les liquides organiques ambiants du patient;

b) au moins un conducteur électrique (204) disposé dans l'autre (202) desdits canaux, ledit conducteur (204) se terminant avec une section transversale prédéterminée (207) à ladite première extrémité dudit organe (101);

c) des moyens d'enrobage (206) pour obturer de manière étanche ledit autre canal (203), lesdits moyens d'enrobage (206) enfermant ledit conducteur (204) tout en exposant ladite section transversale prédéterminée (207); et

des moyens (208) formant une membrane sélectivement perméable recouvrant au moins ladite section transversale prédéterminée exposée (207);

caractérisé en ce que:
ladite ouverture (203) traverse une paroi latérale dudit organe (101) dans la région de ladite première extrémité dudit organe (101), et lesdits moyens d'enrobage (206) obturent également de manière étanche ledit premier canal (201), isolant lesdits moyens (208) formant membrane et ledit conducteur (204) de ladite ouverture (203) et dudit premier canal (201).

2. Un cathéter à deux canaux comprenant un détecteur polarographique selon la revendication 1, caractérisé en ce qu'une partie centrale (209) de l'organe flexible (101) qui sépare les canaux (201, 202) au moins au nombre de deux est évidée vers l'arrière à ladite extrémité dudit organe (101) de façon à pouvoir recevoir un coude formé dans ledit conducteur (204) à l'endroit où il quitte ledit autre canal (202) afin de positionner la terminaison de section transversale prédéterminée (207) au centre de l'extrémité dudit organe flexible (101).

3. Un cathéter à deux canaux comprenant un détecteur polarographique selon la revendication 2, caractérisé en ce que lesdits moyens d'enrobage (206) enferment de manière étanche l'extrémité de ladite partie centrale (209).

4. Un cathéter à deux canaux comprenant un détecteur polarographique selon l'une quelconque des revendications 1 à 3, caractérisé en ce qu'il comporte, en outre, des moyens (103, 104, 105) pour permettre le prélèvement d'échantillons dudit liquide organique ambiant du patient par l'intermédiaire de ladite ouverture (203) et dudit premier canal (201).

5. Un procédé de fabrication d'un cathéter à deux canaux, comprenant un détecteur polarographique, qui consiste:

a) à fabriquer un cathéter à deux canaux flexible (101);

b) à disposer un fil de cathode (204) en métal noble dans un premier canal (202) dudit cathéter;

c) à obturer de manière étanche ledit canal (202) à une première extrémité avec une matière inerte (206), une partie de terminaison dudit fil de section transversale prédéterminée (207) restant exposée; et

d) à recouvrir ladite partie de terminaison de section transversale prédéterminée (207) d'une membrane (208) sélectivement perméable

caractérisé en ce que:

ledit fil (204) se termine à ladite extrémité par une partie que est isolée au vernis (ladite partie de terminaison de section transversale prédéterminée (207) n'étant pas isolée), ladite matière inerte (206) obture, en outre, le second canal (201) dudit cathéter, ladite membrane (208) est formée par enduction par immersion de ladite extrémité de telle sorte que ladite membrane (208) recouvre les parois extérieures dudit cathéter (101) au niveau de ladite extrémité et ladite matière inerte (206), et en ce qu'il est prévu une étape supplémentaire qui consiste à ouvrir (203) ledit second canal (201) à l'extérieur dudit cathéter (101).

6. Un procédé selon la revendication 5, caractérisé en ce que ladite application de revêtement par immersion comprend l'exécution de cycles d'immersion et de séchage successifs effectués en utilisant du poly-hydroxyéthylméthacrylate.

7. Un procédé selon la revendication 5 ou 6, caractérisé en ce que ladite matière inerte est une résine époxyde.

8. Un procédé selon l'une quelconque des revendications 5 à 7, caractérisé en ce qu'une partie centrale (209) du cathéter (101) qui sépare lesdits deux canaux (201, 202) est évidée vers l'arrière à ladite extrémité, et ledit fil (204) est coudé à sa sortie dudit premier passage (202) de façon à positionner la terminaison de section transversale prédéterminée (207) au centre de ladite extrémité dudit cathéter (101).

Fig.1.

Fig.2.